(19) Europäisches Patentamt
European Patent Office
Office européen des brevets

(11) Veröffentlichungsnummer: **0 574 787 A2**

# (12) EUROPÄISCHE PATENTANMELDUNG

(21) Anmeldenummer: **93109121.9**

(22) Anmeldetag: **07.06.93**

(51) Int. Cl.5: **C07D 403/04**, C07D 417/04, C07D 403/14, C07D 417/14, A61K 31/50, A61K 31/54

(30) Priorität: **13.06.92 DE 4219409**
**07.11.92 DE 4237656**

(43) Veröffentlichungstag der Anmeldung:
**22.12.93 Patentblatt 93/51**

(84) Benannte Vertragsstaaten:
**AT BE CH DE DK ES FR GB GR IE IT LI LU NL PT SE**

(71) Anmelder: **MERCK PATENT GmbH**
**Postfach,**
**Frankfurter Strasse 250**
**D-64271 Darmstadt(DE)**

(72) Erfinder: **Dorsch, Dieter, Dr.**
**Königsberger Strasse 17a**
**D-64732 Ober-Ramstadt(DE)**
Erfinder: **Mederski, Werner, Dr.**
**Am Ohlenberg 29**
**D-64390 Erzhausen(DE)**
Erfinder: **Osswald, Mathias, Dr.**
**Katzenelnbogenweg 1**
**D-64673 Zwingenberg(DE)**
Erfinder: **Schelling, Pierre, Prof. dr.**
**Bordenbergweg 17**
**D-64367 Mühltal(DE)**
Erfinder: **Beier, Norbert, Dr.**
**Georgenhäuser Strasse 19**
**D-64354 Reinheim(DE)**
Erfinder: **Lues, Ingeborg, Dr.**
**Katharinenstrasse 2**
**D-64297 Darmstadt(DE)**
Erfinder: **Minck, Klaus-Otto, Dr.**
**Büchestrasse 8**
**W-64372 Ober-Ramstadt(DE)**

## (54) Benzimidazolderivate als Angiotensin II Antagonisten.

(57) Neue Benzimidazolderivate der Formel I

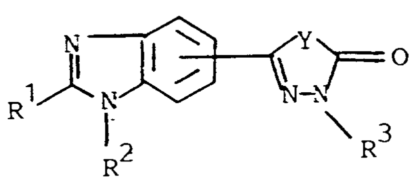

I

worin $R^1$, $R^2$, $R^3$ und Y die in Patentanspruch 1 angegebene Bedeutung haben, sowie deren Salze zeigen angiotensin II-antagonistische Eigenschaften und können zur Behandlung von Hypertension, Aldosteronismus, Herzinsuffizienz und erhöhtem Augeninnendruck sowie von Störungen des Zentralnervensystems verwendet werden.

EP 0 574 787 A2

Die Erfindung betrifft neue Benzimidazolderivate der Formel I

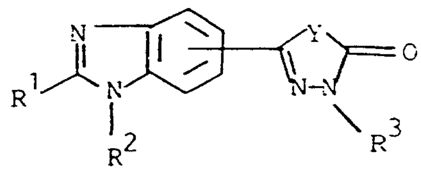

I

worin

- $R^1$ H, A, $C_pH_{2p}$-($C_{3-7}$-Cycloalkyl), OA, SA, Ar oder $Het^1$,
- $R^2$ und $R^3$ jeweils

(1)

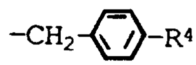

(2)

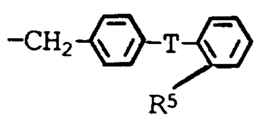

(3)

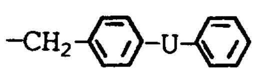

(4) -$C_{1-10}$-Alkyl, -$C_nH_{2n}$-COOR, -$C_nH_{2n}$-CN, -$C_mH_{2m}$-Ar, -$C_mH_{2m}$-$Het^2$, -$C_nH_{2n}$-1H-5-Tetrazolyl, -$C_nH_{2n}$-CH = CH-Ar, -$C_nH_{2n}$-CO-N$(R)_2$, -$C_mH_{2m}$-CO-R, -$C_mH_{2m}$-CO-Ar, -$C_nH_{2n}$-O-CON$(R)_2$ oder -$C_nH_{2n}$-NR-CO-N$(R)_2$, oder

(5) H,

- Y -$C(R)_2$-$C(R)_2$-, -CR = CR- oder -$C(R)_2$-S-,

die Reste R unabhängig voneinander H oder A,

- $R^4$ COOR, CN oder 1H-5-Tetrazolyl,
- $R^5$ COOR, CN, $NO_2$, $NH_2$, $NHCOCF_3$, $NHSO_2CF_3$ oder 1H-5-Tetrazolyl,
- T fehlt, -NR-CO-, -CO-NR- oder -CH = CH-,
- U -CH = C(COOR)-, -CH = C(CN)-, -CH = C(1H-5-Tetrazolyl)-, -O-CH(COOR)- oder -NR-CH-(COOR)-,
- m und p jeweils 0, 1, 2, 3, 4, 5, 6, 7, 8, 9 oder 10,
- n 1, 2, 3, 4, 5, 6, 7, 8, 9 oder 10,
- A Alkyl mit 1-6 C-Atomen,
- Ar eine unsubstituierte oder eine ein- oder zweifach durch A, Hal, $CF_3$, OH, OA, COOH, COOA, CN, $NO_2$, $NH_2$, NHA und/oder $N(A)_2$ substituierte Phenyl- oder Naphthylgruppe,
- $Het^1$ eine unsubstituierte oder eine durch A, $C_{1-7}$-Alkanoyl oder COOA monosubstituierte Indolyl-, 2,3-Dihydroindolyl-, Benzofuryl-, 2,3-Dihydrobenzofuryl-, Benzothienyl- oder 2,3-Dihydrobenzothienyl-gruppe,
- $Het^2$ einen unsubstituierten oder durch A substituierten fünf- oder sechsgliedrigen heteroaromatischen Rest mit 1 bis 3 N-, O- und/oder S-Atomen, der auch mit einem Benzol- oder Pyridinring kondensiert sein kann und
- Hal F, Cl, Br oder I bedeuten,

mit der Maßgabe, daß

1. Y nur dann $-C(R)_2-C(R)_2-$ oder $-CR=CR-$ bedeutet, wenn $R^1$ von $-C_pH_{2p}-(C_{3-7}$-Cycloalkyl) und/oder wenn $R^3$ von H verschieden ist,

2. mindestens einer der Reste $R^2$ und $R^3$ eine der Bedeutungen (1), (2) oder (3) hat,

sowie ihre Salze.

Ähnliche Verbindungen sind aus EP-A1-0 468 470 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher zur Behandlung der angiotensin II-abhängigen Hypertension, des Aldosteronismus und der Herzinsuffizienz sowie von Störungen des Zentralnervensystems eingesetzt werden. Diese Wirkungen können nach üblichen in vitro- oder in vivo-Methoden ermittelt werden, wie sie z.B. in der EP-A1-0 468 470, in der US-PS 4 880 804 und in der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Die Verbindungen der Formel I können als Arzneimittelwirkstoffe in der Human- und Veterinärmedizin eingesetzt werden, insbesondere zur Prophylaxe und/oder Therapie von Herz-, Kreislauf- und Gefäßkrankheiten, vor allem von Hypertonie, Herzinsuffizienz und Hyperaldosteronismus, ferner von Hypertrophie und Hyperplasie der Blutgefäße und des Herzens, Angina pectoris, Herzinfarkten, Schlaganfall, Restenosen nach Angioplastie oder By-pass-Operationen, Arteriosklerose, erhöhtem Augeninnendruck, Glaukomen, macularer Degeneration, Hyperurikämie, Nierenfunktionsstörungen, z.B. Nierenversagen, Nephropathia diabetica, Retinopathia diabetica, Psoriasis, angiotensin II-vermittelten Störungen in weiblichen Fortpflanzungsorganen, Wahrnehmungsstörungen, z.B. Demenz, Amnesie, Gedächnisfunktionsstörungen, Angstzuständen, Depression und/oder Epilepsie.

Gegenstand der Erfindung sind die Verbindungen der Formel I, ihre Salze sowie ein Verfahren zur Herstellung dieser Verbindungen und ihrer Salze, dadurch gekennzeichnet, daß man

(a) eine Verbindung der Formel II

E-Z II

worin

E Cl, Br, I oder eine freie oder reaktionsfähig funktionell abgewandelte OH-Gruppe und

Z $R^2$ oder $R^3$, jedoch nicht H, bedeuten,

mit einer Verbindung der Formel III

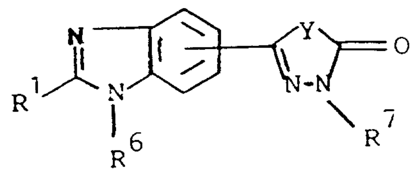

III

worin

$R^6$ $R^2$ (falls Z $R^3$ ist) und

$R^7$ $R^3$ (falls Z $R^2$ ist) bedeuten,

mindestens einer der Reste $R^6$ und $R^7$ jedoch H bedeutet und $R^1$ und Y die in Anspruch 1 angegebene Bedeutung haben, umsetzt,

oder

(b) eine Verbindung der Formel IV

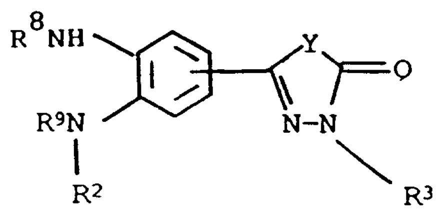

IV

worin

$R^8$ $R_1$-CO oder H, und

$R^9$ H (falls $R^8$ $R^1$-CO ist) oder $R^1$-CO (falls $R^8$ H ist)

bedeuten,

und $R^1$, $R^2$, $R^3$ und Y die in Anspruch 1 angegebenen Bedeutungen haben,

mit einem cyclisierenden Mittel behandelt,

oder

(c) zur Herstellung einer Verbindung der Formel I, worin T -NR-CO- oder -CO-NR- bedeutet, eine Verbindung der Formel V

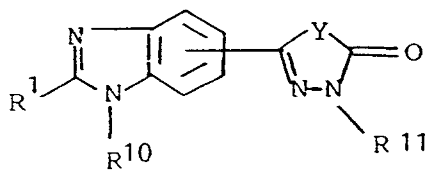

V

worin

$R^{10}$ $R^2$ oder

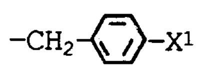

$R^{11}$ $R^3$ (falls

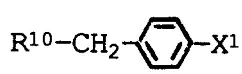

bedeutet) oder

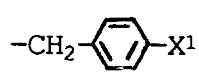

(falls $R^{10}$ $R^2$ bedeutet), und

$X^1$ $NH_2$ oder COOH bedeuten, und

$R^1$ und Y die in Anspruch 1 angegebenen Bedeutungen haben,

oder ein reaktionsfähiges Derivat dieser Verbindung mit einer Verbindung der Formel VI

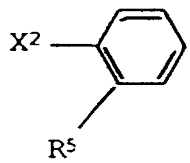

VI

worin

$X^2$ COOH (falls $X^1$ $NH_2$ ist) oder $NH_2$ (falls $X^1$ COOH ist) bedeutet und

$R^5$ die in Anspruch 1 angegebene Bedeutung hat,

oder mit einem reaktionsfähigen Derivat dieser Verbindung umsetzt,

oder daß man eine Verbindung der Formel I aus einem ihrer funktionellen Derivate durch Behandeln mit einem solvolysierenden oder hydrogenolysierenden Mittel in Freiheit setzt,

und/oder daß man in einer Verbindung der Formel I einen oder mehrere Rest(e) $R^2$ und/oder $R^3$ in einen oder mehrere andere Reste $R^2$ und/oder $R^3$ umwandelt und/oder eine Verbindung der Formel I, worin Y -CHR-CHR- bedeutet, durch Behandeln mit einem dehydrierenden Mittel in eine Verbindung der Formel I, worin Y -CR=CR- bedeutet, überführt und/oder eine Base oder Säure der Formel I in eines ihrer Salze umwandelt.

Vor- und nachstehend haben die Reste bzw. Parameter $R^1$ bis $R^{11}$, R, T, U, m, n, p, $X^1$, $X^2$, Y, A, Ar, $Het^1$, $Het^2$, Hal, E und Z die bei den Formeln I bis V angegebenen Bedeutungen, falls nicht ausdrücklich etwas anderes angegeben ist.

In den vorstehenden Formeln bedeutet A insbesondere Alkyl mit 1-6, vorzugsweise 1, 2, 3, oder 4 C-Atomen, vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.

Dementsprechend ist der Rest OA vorzugsweise Methoxy, weiterhin Ethoxy, Propoxy, Isopropoxy, Butoxy, Isobutoxy, sek.-Butoxy oder tert.-Butoxy, der Rest SA bevorzugt Methylthio, weiterhin Ethylthio. Die Gruppe COOA ist bevorzugt Methoxycarbonyl oder Ethoxycarbonyl, ferner Propyloxycarbonyl, Isopropyloxycarbonyl, Butyloxycarbonyl, Isobutyloxycarbonyl. Die Gruppe NHA ist bevorzugt Methylamino oder Ethylamino. Die Gruppe $N(A)_2$ ist bevorzugt Dimethylamino oder Diethylamino.

Cycloalkyl ist bevorzugt Cyclopropyl, ferner Cyclobutyl, Cyclopentyl, Cyclohexyl oder Cycloheptyl, aber auch z.B. 1- oder 2-Methylcyclopropyl, 1-, 2- oder 3-Methylcyclopentyl, 1-, 2-, 3- oder 4-Methylcyclohexyl.

Hal bedeutet vorzugsweise F, Cl oder Br, aber auch J.

Der Rest Ar ist vorzugsweise eine unsubstituierte Phenylgruppe, weiterhin bevorzugt eine in p-Stellung monosubstituierte, aber auch in o- oder m-Stellung monosubstituierte Phenylgruppe. Bevorzugte Substituenten sind OA, COOH, COOA und $NO_2$. Dementsprechend ist Ar bevorzugt Phenyl, o-, m- oder (insbesondere) p-Methoxyphenyl, o-, m- oder (insbesondere) p-Carboxyphenyl, o-, m- oder (insbesondere) p-Methoxycarbonylphenyl, o-, m- oder (insbesondere) p-Ethoxycarbonylphenyl, o-, m- oder (insbesondere) p-Nitrophenyl, ferner bevorzugt o-, m- oder (insbesondere) p-Aminophenyl, o-, m- oder (insbesondere) p-Dimethylaminophenyi, o-, m- oder (insbesondere) p-Diethylaminophenyl, o-, m- oder p-Tolyl, o-, m- oder p-Trifluormethylphenyl, o-, m- oder p-Hydroxyphenyl, o-, m- oder p-Fluorphenyl, o-, m- oder p-Chlorphenyl, o-, m- oder p-Bromphenyl, o-, m- oder p-Iodphenyl, o-, m- oder p-Cyan-phenyl, o-, m- oder p-Methylaminophenyl, 1- oder 2-Naphthyl. Der Rest $Het^1$ ist vorzugsweise 2,3-Dihydro-1-methoxycarbonyl-2-, -3-, -4-, -5-, -6- oder -7-indolyl, 2,3-Dihydro-1-ethoxycarbonyl-2-,-3-, -4-, -5-, -6- oder -7-indolyl; $Het^1$ ist ferner bevorzugt unsubstituiertes 1-, 2-, 3-, 4-, 5-, 6- oder 7-Indolyl, 2-, 3-, 4-, 5-, 6- oder 7-Benzofuryl, 2,3-Dihydro- 2-, -3-, -4-, -5-, -6- oder -7-benzofuryl, 2-, 3-, 4-, 5-, 6- oder 7-Benzothienyl, 2,3-Dihydro-2-, -3-, -4-, -5-, -6- oder -7-benzothienyl, wobei diese Reste in einer der freien Stellen durch A (bevorzugt Methyl), $C_{1-7}$-Alkanoyl (bevorzugt Acetyl) oder COOA (bevorzugt Methoxycarbonyl) oder Ethoxycarbonyl substituiert sein können.

$Het^2$ ist vorzusweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder -5-yl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2-1,3-oxadiazolyl, 2-,

3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolyl, 2-, 4-, 5-, 6-, 7- oder 8-Chinazolyl, 1H-1-, -2-, -5-, -6- oder -7-Imidazo[4,5-b]pyridyl, 3H-2-, -3-, -5-, -6- oder -7-Imidazo[4,5-b]pyridyl, 1H-1-, -2-, -4-, -6- oder -7-Imidazo[4,5-c]pyridyl, 3H-2-, -3-, -4-, -6- oder -7-Imidazo-[4,5-c]pyridyl.

In den Begriff "$Het^2$" eingeschlossen sind auch die homologen Reste, in denen der heteroaromatische Ring durch eine oder mehrere, vorzugsweise 1 oder 2, A-Gruppen, vorzugsweise Methyl- und/oder Ethylgruppen substituiert ist, z. B. 3-, 4- oder 5-Methyl-2-furyl, 2-, 4- oder 5-Methyl-3-furyl, 2,4-Dimethyl-3-furyl, 3-, 4- oder 5-Methyl-2-thienyl, 3-Methyl-5-tert.-butyl-2-thienyl, 2-, 4- oder 5-Methyl-3-thienyl,2- oder 3-Methyl-1-pyrrolyl, 1-, 3-, 4- oder 5-Methyl-2-pyrrolyl, 3,5-Dimethyl-4-ethyl-2-pyrrolyl, 2-, 4- oder 5-Methyl-1-imidazolyl, 4-Methyl-5-pyrazolyl, 4- oder 5-Methyl-3-isoxazolyl, 3- oder 5-Methyl-4-isoxazolyl, 3- oder 4-Methyl-5-isoxazolyl, 3,4-Dimethyl-5-isoxazolyl, 4- oder 5-Methyl-2-thiazolyl, 4- oder 5-Ethyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 3-, 4-, 5-, 6- oder 7-Methyl-2-benzofuryl, 2-Ethyl-3-benzofuryl, 3-, 4-, 5-, 6- oder 7-Methyl-2-benzothienyl, 3-Ethyl-2-benzothienyl, 1-, 2-, 4-, 5-, 6- oder 7-Methyl-3-indolyl, 1-Methyl-5- oder 6-benzimidazolyl, 1-Ethyl-5- oder 6-benzimidazolyl.

Der Rest T fehlt vorzugsweise; weiterhin ist T bevorzugt -NH-CO-, $-N(CH_3)-CO-$, -CO-NH-, $-CO-N(CH_3)-$ oder -CH=CH-.

Der Rest U ist vorzugsweise -CH=C(CN)- oder -CH=C(1H-5-Tetrazolyl)-, ferner bevorzugt -CH=C-(COOH)-, $-CH=C(COOCH_3)-$, $-CH=C(COOC_2H_5)-$, -O-CH(COOH)-, $-O-CH(COOCH_3)-$, $-O-CH(COOC_2H_5)-$, -NH-CH(COOH)-, $-N(CH_3)-CH(COOH)-$, $-NH-CH(COOCH_3)-$, $-N(CH_3)-CH(COOCH_3)-$, $-NH-CH(COOC_2H_5)-$ oder $-N(CH_3)-CH(COOC_2H_5)-$.

Der Rest Y ist vorzugsweise $-CH(CH_3)-CH_2-$ oder $-C(CH_3)=CH-$.

Die Reste R stehen unabhängig voneinander bevorzugt für H, $CH_3$ oder $C_2H_5$.

Der Rest $R^1$ ist vorzugsweise A oder Cycloalkyl, insbesondere Butyl oder Cyclopropyl, ferner bevorzugt Propyl, Pentyl oder Hexyl, weiterhin bevorzugt H, Cyclopropylmethyl, p-Methoxyphenyl oder 2,3-Dihydro-1-methoxycarbonyl-indolyl.

Der Rest $R^2$ ist vorzugsweise H, 2'-Cyan-biphenylyl-4-methyl, 2'-Carboxy-biphenylyl-4-methyl, 2'-(1H-5-Tetrazolyl)-biphenylyl-4-methyl oder p-(2-Cyan-2-phenyl-vinyl)-benzyl.

Der Rest $R^3$ ist vorzugsweise H, Carboxymethyl, o-, m- oder (insbesondere) p-Carboxybenzyl, 2'-Cyan-biphenylyl-4-methyl, 2'-Carboxy-biphenylyl-4-methyl oder 2'-(1H-Tetrazolyl)-biphenylyl-4-methyl.

Der Rest $R^4$ ist vorzugsweise COOH, ferner bevorzugt $COOCH_3$, $COOC_2H_5$, CN oder 1H-5-Tetrazolyl.

Der Rest $R^5$ ist vorzugsweise COOH, $COOCH_3$, $COOC_2H_5$, CN oder 1H-5-Tetrazolyl.

Der Parameter m ist vorzugsweise 0, 1 oder 2, der Parameter p ist vorzugsweise 0 oder 1, der Parameter n ist vorzugsweise 1 oder 2. Die Gruppe $C_mH_{2m}$ steht dabei bevorzugt für $-(CH_2)_m-$, insbesondere für $-CH_2-$ oder $-CH_2CH_2-$, die Gruppe $C_pH_{2p}$ bevorzugt für $-(CH_2)_p-$, insbesondere für $-CH_2-$, die Gruppe $C_nH_{2n}$ bevorzugt für $-(CH_2)_n-$, insbesondere für $-CH_2-$ oder $-CH_2CH_2-$.

Die Verbindungen der Formel I können ein oder mehrere chirale Zentren besitzen und daher in verschiedenen - optisch-aktiven oder optisch-inaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Id ausgedrückt werden, die der Formel I entsprechen und worin die nicht näher bezeichneten Reste die bei Formel I angegebenen Bedeutungen haben, worin jedoch

- in Ia $R^1$ H, A, Cyclopropyl, p-Methoxyphenyl oder 2,3-Dihydro-1-methoxycarbonyl-indolyl bedeutet;
- in Ib $R^1$ A bedeutet;
- in Ic $R^2$ H, 2'-Cyan-biphenylyl-4-methyl, 2'-Carboxybiphenylyl-4-methyl, 2'-(1H-5-Tetrazolyl)-biphenylyl-4-methyl oder p-(2-Cyan-2-phenylvinyl)-benzyl bedeutet;
- in Id $R^1$ A und
  $R^2$ H, 2'-Cyan-biphenylyl-4-methyl, 2'-Carboxybiphenylyl-4-methyl, 2'-(1H-5-Tetrazolyl)-biphenylyl-4-methyl oder p-(2-Cyan-2-phenylvinyl)-benzyl bedeuten.

Weiterhin sind bevorzugt Verbindungen der Formeln:

Ie sowie Iae, Ibe, Ice und Ide, die den Formeln I sowie Ia, Ib, Ic und Id entsprechen, worin jedoch zusätzlich $R^3$ H, Carboxymethyl, Carboxybenzyl, 2'-Cyan-biphenylyl-4-methyl, 2'-Carboxy-biphenylyl-4-methyl oder 2'-(1H-5-Tetrazolyl)-biphenylyl-4-methyl bedeutet;

If sowie Iaf, Ibf, Icf und Idf, die den Formeln I sowie Ia, Ib, Ic und Id entsprechen, worin jedoch zusätzlich $R^3$ H bedeutet;

Ig sowie Iag, Ibg, Icg und Idg, die den Formeln I sowie Ia, Ib, Ic und Id entsprechen, worin jedoch zusätzlich $R^3$ Carboxymethyl bedeutet.

Insbesondere sind bevorzugt Verbindungen aller vorstehend genannten Formeln, in denen zusätzlich Y $-CH(CH_3)-CH_2-$ oder $-C(CH_3)=CH-$ bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; insbesondere aber in der EP-A2-0 468 470 und in der US-PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können vorzugsweise erhalten werden, indem man Verbindungen der Formel II mit Verbindungen der Formel III umsetzt.

In den Verbindungen der Formel II bedeutet E vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy).

Die Umsetzung von II mit III erfolgt zweckmäßig, indem man zunächst III durch Behandeln mit einer Base in ein Salz umwandelt, z.B. mit einem Alkalimetallalkoholat wie $CH_3ONa$ oder K-tert.-Butylat in einem Alkohol wie $CH_3OH$, in einem Ether wie Tetrahydrofuran (THF) oder in einem Amid wie Dimethylformamid (DMF) oder mit einem Alkalimetallhydrid wie NaH oder einem Alkalimetallalkoholat in DMF, und dieses dann in einem inerten Lösungsmittel, z.B. einem Amid wie DMF oder Dimethylacetamid oder einem Sulfoxid wie Dimethylsulfoxid (DMSO), mit II umsetzt, zweckmäßig bei Temperaturen zwischen -20 und 100°, vorzugsweise zwischen 10 und 30°. Als Basen eignen sich auch Alkalimetallcarbonate wie $Na_2CO_3$ oder $K_2CO_3$ oder Alkalimetall-hydrogencarbonate wie $NaHCO_3$ oder $KHCO_3$.

Verwendet man eine Verbindung der Formel III, worin $R^6 = R^7 = H$ sind, so erhält man bei der Reaktion mit II in der Regel Gemische, die jedoch leicht trennbar sind, z.B. chromatographisch. Das Mengenverhältnis und die Art der Produkte können durch Abwandlung der Reaktionsbedingungen gesteuert werden. So entsteht beim Einsatz äquimolarer Mengen II und III vorwiegend das am Benzimidazol-N-atom substituierte Produkt, wenn man eine äquimolare Menge der Base verwendet; dagegen entsteht vorwiegend das in 1-Stellung des Pyridazin-on- bzw. Thiopyridazinonrings substituierte Produkt, wenn man mit einem Überschuß der Base arbeitet.

Die Verbindungen der Formel I sind weiterhin durch Cyclisierung von Verbindungen der Formel IV erhältlich. Diese Cyclisierung gelingt zweckmäßig durch Erhitzen mit Polyphosphorsäure, Essigsäure oder Diglyme auf Temperaturen zwischen etwa 80 und 180°, vorzugsweise zwischen 120 und 160°.

Säureamide der Formel I (T = -NR-CO- oder -CO-NR-) sind ferner erhältlich durch Umsetzung von Verbindungen der Formel V (oder ihrer reaktionsfähigen Derivate) mit Verbindungen der Formel VI (oder ihrer reaktionsfähigen Derivate).

Als reaktionsfähige Derivate der Carbonsäuren der Formel V und VI ($X^1$ bzw. $X^2$ = COOH) eignen sich vorteilhaft die entsprechenden Chloride, Bromide oder Anhydride. Die Umsetzung erfolgt zweckmäßig in Gegenwart eines inerten Lösungsmittels, z.B. eines halogenierten Kohlenwasserstoffs wie Dichlormethan, Chloroform, Trichlorethen oder 1,2-Dichlorethan oder eines Ethers wie THF oder Dioxan, bei Temperaturen zwischen 0 und 150°, vorzugsweise zwischen 20 und 80°. Setzt man Säurehalogenide um, so empfiehlt sich der Zusatz einer Base, z.B. eines tertiären Amins wie Triethylamin, Pyridin oder 4-Dimethylaminopyridin.

Weiterhin kann man eine Verbindung der Formel I durch Solvolyse (z.B. Hydrolyse) oder Hydrogenolyse aus einem ihrer funktionellen Derivate in Freiheit setzen.

So kann man Carbonsäuren der Formel I, worin U -O-CH(COOH), -NH-CH(COOH), -NA-CH(COOH) oder -CH=C(COOH) bedeutet, erhalten durch Verseifung entsprechender Alkylester, z.B. mit NaOH oder KOH in wässeriger Lösung mit oder ohne Zusatz eines inerten organischen Lösungsmittels wie Methanol, Ethanol, THF oder Dioxan bei Temperaturen zwischen 0 und 100°, oder durch Hydrogenolyse entsprechender Benzylester, z.B. an Pd-Kohle bei Drucken zwischen 1 und 200 bar und bei Temperaturen zwischen 0 und 100° in einem der angegebenen inerten Lösungsmittel.

Ferner ist es möglich, nach einer der angegebenen Methoden eine Verbindung herzustellen, die der Formel I entspricht, aber an Stelle einer 5-Tetrazolylgruppe eine in 1-Stellung (oder 2-Stellung) funktionell abgewandelte (durch eine Schutzgruppe geschützt) 1H- (oder 2H)-5-Tetrazolylgruppe enthält, und diese Schutzgruppe zum Schluß abzuspalten. Als Schutzgruppen eignen sich beispielsweise: Triphenylmethyl, abspaltbar mit HCl oder Ameisensäure in einem inerten Lösungsmittel oder Lösungsmittelgemisch, z.B.

Ether/Dichlormethan/Methanol; 2-Cyanethyl, abspaltbar mit NaOH in Wasser/THF; p-Nitrobenzyl, abspaltbar mit $H_2$/Raney-Nickel in Ethanol (vgl. EP-A2-0 291 969).

Die Ausgangsstoffe, insbesondere diejenigen der Formeln II und VI, sind teilweise bekannt. Falls sie nicht bekannt sind, können sie nach bekannten Methoden in Analogie zu bekannten Stoffen hergestellt werden.

Verbindungen der Formel III sind z.B. erhältlich durch Reaktion von Carbonsäuren der Formel $R^1$-COOH mit Verbindungen der Formel VII

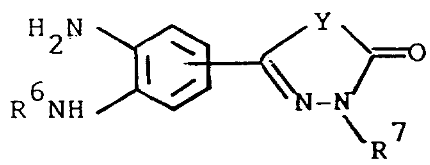

VII

in Gegenwart von Polyphosphorsäure.

Verbindungen der Formel IV sind z.B. erhältlich durch Umsetzung von Verbindungen der Formel VIII

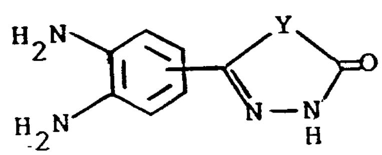

VIII

worin jedoch die eine Aminogruppe durch eine Aminoschutzgruppe (z.B. Benzyl, A-O-CO- oder Benzyloxycarbonyl) geschützt ist, mit Verbindungen der Formel II sowie nachfolgende Abspaltung der Schutzgruppe und Reaktion mit Säuren der Formel $R^1$-COOH oder deren funktionellen Derivaten; sie werden in der Regel nicht isoliert, sondern entstehen in situ bei der letztgenannten Umsetzung. Anstelle der Säuren der Formel $R^1$-COOH können auch die entsprechenden Aldehyde in Gegenwart eines Oxydationsmittels wie $Na_2S_2O_5$ eingesetzt werden. Als funktionelles Derivat der Ameisensäure ($R^1$ = H) eignet sich auch 1,3,5-Triazin.

Verbindungen der Formel V können durch Reaktion von III mit Benzylchloriden der Formel Cl-$CH_2$-p-$C_6H_4$-$X^3$) (worin $X^3$ eine geschützte $NH_2$- oder COOH-Gruppe bedeutet) und nachfolgende Abspaltung der Schutzgruppe hergestellt werden.

Es ist ferner möglich, eine Verbindung der Formel I in eine andere Verbindung der Formel I umzuwandeln, indem man einen oder mehrere der Reste $R^2$ und/oder $R^3$ in andere Reste $R^2$ und/oder $R^3$ umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Halogenatome (z.B. durch Reaktion mit Kupfer(I)cyanid) durch CN-Gruppen ersetzt und/oder Nitrilgruppen zu COOH-Gruppen oder zu $CONH_2$-Gruppen hydrolysiert oder mit Derivaten der Stickstoffwasserstoffsäure, z.B. Natriumazid in N-Methylpyrrolidon oder Trimethylzinnazid in Toluol, zu Tetrazolylgruppen umsetzt.

So kann man beispielsweise freie Aminogruppen in üblicher Weise mit einem Säurechlorid oder -anhydrid acylieren oder freie Hydroxy- und/oder NH-Gruppen mit einem unsubstituierten oder substituierten Alkylhalogenid oder mit Aldehyden wie Formaldehyd in Gegenwart eines Reduktionsmittels wie $NaBH_4$ oder Ameisensäure alkylieren, zweckmäßig in einem inerten Lösungsmittel wie Dichlormethan oder THF und/ oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z. B. eine Verbindung der Formel I, die eine COOA-Gruppe enthält, in die entsprechende Verbindung der Formel I umgewandelt werden, die stattdessen eine COOH-Gruppe enthält. Estergruppen können z.B. mit NaOH oder KOH in Methanol, Wasser, Wasser-THF oder Wasser-Dioxan bei Temperaturen zwischen 0 und 100° verseift werden.

Umsetzung von Nitrilen der Formel I ($R^2$ oder $R^3$ = -$C_nH_{2n}$-CN; oder $R^4$ oder $R^5$ = CN) mit Derivaten der Stickstoffwasserstoffsäure führt zu Tetrazolen der Formel I ($R^2$ oder $R^3$ = -$C_nH_{2n}$-1H-5-Tetrazolyl; $R^4$

oder $R^5$ = 1H-5-Tetrazolyl). Bevorzugt verwendet man Trialkylzinnazide wie Trimethylzinnazid in einem inerten Lösungsmittel, z.B. einem aromatischen Kohlenwasserstoff wie Toluol bei Temperaturen zwischen 20 und 150°, vorzugsweise zwischen 80 und 140°, oder Natriumazid in N-Methylpyrrolidon bei Temperaturen zwischen etwa 100 und 200°. Anschließend wird die Trialkylzinn-Gruppe abgespalten, entweder durch Behandeln mit Salzsäure, z.B. in Dioxan, oder mit Alkali, z.B. in Ethanol/Wasser, oder mit Ameisensäure, z.B. in Methanol, oder durch Chromatographie an einer Kieselgel-Säule, z.B. mit Ethylacetat/Methanol.

Ferner kann man eine Verbindung der Formel I, worin Y -CHR-CHR- bedeutet, zu einer Verbindung der Formel I, worin Y -CR=CR- bedeutet, dehydrieren, vorzugsweise mit Natrium-3-nitrobenzolsulfonat in wässerig-alkalischer Lösung bei Temperaturen zwischen 0 und 100°. Andere geeignete Dehydrierungsmittel sind z.B. Brom in Essigsäure, $MnO_2$ oder $SOCl_2$.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel, z.B. Ethanol, und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I, die COOH- oder Tetrazolylgruppen enthalten, mit Basen (z.B. Natrium- oder Kaliumhydroxid oder -carbonat) in die entsprechenden Metall-, insbesondere Alkalimetall- oder Erdalkalimetall-, oder in die entsprechenden Ammoniumsalze umgewandelt werden. Die Kaliumsalze sind besonders bevorzugt.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff-(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vor zugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgas oder Treibgasgemisch (z.B. Kohlenwasserstoffen wie Propan oder Butan oder Fluorkohlenwasserstoffen wie Heptafluorpropan) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, insbesondere aber in Analogie zu den in der US-PS 4 880 804 beschriebenen Verbindungen verabreicht, vorzugsweise in Dosierungen zwischen etwa 1 mg und 1 g, insbesondere zwischen 50 und 500 mg pro Dosierungseinheit. Die tägliche Dosierung liegt vorzugsweise zwischen etwa 0,1 und 100 mg/kg, insbesondere 1 und 50 mg/kg Körpergewicht. Die spezielle Dosis für jeden bestimmten Patienten hängt jedoch von den verschiedensten Faktoren ab, beispielsweise von der Wirksamkeit der

eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation. FAB = Peak im Massenspektrum, erhalten nach der "Fast Atom Bombardment"-Methode. Rf = Rf-Wert, dünnschichtchromatographisch an Kieselgel mit Ethylacetat/Methanol 95:5.

**Beispiel 1**

(a) Eine Lösung von 2,84 g 2-Butyl-5(1,4,5,6-tetrahydro-4-methyl-6-oxo-3-pyridazinyl)-benzimidazol ["IIIa"; F. 193°; erhältlich durch Reaktion von 3-(3,4-Diaminophenyl)-1,4,5,6-tetrahydro-4-methyl-6-pyridazinon mit Valeriansäure und nachfolgende Cyclisierung mit Essigsäure analog Beispiel 3] in 70 ml THF wird mit 1,12 g K-tert.-Butylat versetzt, 30 Min. bei 20° gerührt und anschließend mit 3,15 g 4'-Brommethyl-biphenyl-2-carbonsäuremethylester (IIa) versetzt. Man rührt 28 Std. bei 20°, dampft ein und arbeitet wie üblich auf (Ethylacetat/gesättigte $NH_4Cl$-Lösung; Chromatographie an Kieselgel mit Ethylacetat/Methanol 99:1). Nacheinander erhält man 2-Butyl-1-(2'-methoxycarbonyl-biphenylyl-4-methyl)- 6-(1,4,5,6-tetrahydro-4-methyl-6-oxo-3-pyridazinyl)-benzimidazol (Öl), dann 2-Butyl-1-(2'-methoxycarbonyl-biphenylyl-4-methyl)-5-(1,4,5,6-tetrahydro-4-methyl-6-oxo-3-pyridazinyl)-benzimidazol (Öl).

(b) Aus den nach (a) erhaltenen Produkten erhält man durch Reaktion mit Bromessigsäuremethylester nach der unter (a) beschriebenen Methodik 2-Butyl-1-(2'-methoxycarbonyl-biphenylyl-4-methyl)-6-(1,4,5,6-tetrahydro-1-methoxycarbonylmethyl-4-methyl-6-oxo-3-pyridazinyl)-benzimidazol (Öl; FAB 581) und 2-Butyl-1-(2'-methoxycarbonyl-biphenylyl-4-methyl)-5-(1,4,5,6-tetrahydro-1-methoxycarbonylmethyl-4-methyl-6-oxo-3-pyridazinyl)-benzimidazol (Öl; FAB 581).

(c) Eine Lösung von 1 g des erstgenannten nach (b) erhaltenen Diesters in 6 ml 2 N wässeriger Natronlauge und 30 ml Dioxan wird 48 Std. bei 20° gerührt. Man konzentriert die Lösung, nimmt den Rückstand in Wasser auf, säuert mit 1 N Salzsäure an und filtriert das so erhaltene 2-Butyl-1-(2'-carboxy-biphenylyl-4-methyl)-5-(1-carboxymethyl-1,4,5,6-tetrahydro-4-methyl-6-oxo-3-pyridazinyl)-benzimidazol ab; FAB 553; F. 155°.

Analog erhält man durch Verseifung des zweitgenannten nach (b) erhaltenen Diesters das 2-Butyl-1-(2'-carboxybiphenylyl-4-methyl)-6-(1-carboxymethyl-1,4,5,6-tetrahydro-4-methyl-6-oxo-3-pyridazinyl)-benzimidazol; FAB 553; F. 143°.

Analog erhält man:

aus IIIa und 4,-Brommethyl-2-cyan-biphenyl (IIb):
2-Butyl-1-(2'-cyan-biphenylyl-4-methyl)-5-(1,4,5,6-tetrahydro-4-methyl-6-oxo-3-pyridazinyl)-benzimidazol und 2-Butyl-1-(2'-cyan-biphenylyl-4-methyl)-6-(1,4,5,6-tetrahydro-4-methyl-6-oxo-3-pyridazinyl)-benzimidazol;

aus 2-Butyl-5-(3,6-dihydro-2-oxo-2H-1,3,4-thiadiazin-5-yl)-benzimidazol (IIIb) und IIa:
2-Butyl-1-(2'-methoxycarbonyl-biphenylyl-4-methyl)-5-(3,6-dihydro-2-oxo-2H-1,3,4-thiadiazin-5-yl)-benzimidazol und 2-Butyl-1-(2'-methoxycarbonyl-biphenylyl-4-methyl)-6-(3,6-dihydro-2-oxo-2H-1,3,4-thiadiazin-5-yl)-benzimidazol;

aus IIIb und IIb:
2-Butyl-1-(2'-cyan-biphenylyl-4-methyl)-5-(3,6-dihydro-2-oxo-2H-1,3,4-thiadiazin-5-yl)-benzimidazol und 2-Butyl-1-(2'-cyan-biphenylyl-4-methyl)-6-(3,6-dihydro-2-oxo-2H-1,3,4-thiadiazin-5-yl)-benzimidazol;

aus IIIa und Bromessigsäuremethylester:
2-Butyl-1-methoxycarbonylmethyl-5-(1,4,5,6-tetrahydro-4-methyl-6-oxo-3-pyridazinyl)-benzimidazol und 2-Butyl-1-methoxycarbonyl-methyl-6-(1,4,5,6-tetrahydro-4-methyl-6-oxo-3-pyridazinyl)-benzimidazol;

aus 2-Butyl-5-(3,6-dihydro-6-methyl-2-oxo-2H-1,3,4-thiadiazin-5-yl)-benzimidazol und IIb:
2-Butyl-1-(2'-cyan-biphenylyl-4-methyl)-5-(3,6-dihydro-6-methyl-2-oxo-2H-1,3,4-thiadiazin-5-yl)-benzimidazol und 2-Butyl-1-(2'-cyan-biphenylyl-4-methyl)-6-(3,6-dihydro-6-methyl-2-oxo-2H-1,3,4-thiadiazin-5-yl)-benzimidazol.

Analog erhält man die nachstehenden Benzimidazole:

2-Butyl-1-(2'-cyan-biphenylyl-4-methyl)-5-(1,4,5,6-tetrahydro-1-methoxycarbonylmethyl-4-methyl-6-oxo-3-pyridazinyl)-2-Butyl-1-(2'-cyan-biphenylyl-4-methyl)-6-(1,4,5,6-tetrahydro-1-methoxycarbonylmethyl-4-methyl-6-oxo-3-pyridazinyl)-2-Butyl-1-(2'-cyan-biphenylyl-4-methyl)-5-(3,6-dihydro-1-methoxycarbonylmethyl-2-oxo-2H-1,3,4-thiadiazin-5-yl)-2-Butyl-1-(2'-cyan-biphenylyl-4-methyl)-6-(3,6-dihydro-1-methoxycarbonylmethyl-2-oxo-2H-1,3,4-thiadiazin-5-yl)-;
(mit IIb):
2-Butyl-5-[1-(2'-cyan-biphenylyl-4-methyl)-1,4,5,6-tetrahydro-4-methyl-6-oxo-3-pyridazinyl]-1-methoxycarbonylmethyl-2-Butyl-6-[1-(2'-cyan-biphenylyl-4-methyl)-1,4,5,6-tetrahydro-4-methyl-6-oxo-3-pyridazinyl]-1-methoxycarbonylmethyl-;
(mit o-Brommethyl-benzoesäuremethylester):
2-Butyl-1-(2'-cyan-biphenylyl-4-methyl)-5-(1,4,5,6-tetrahydro-1-o-methoxycarbonylbenzyl-4-methyl-6-oxo-3-pyridazinyl)-2-Butyl-1-(2'-cyan-biphenylyl-4-methyl)-6-(1,4,5,6-tetrahydro-1-o-methoxycarbonylbenzyl-4-methyl-6-oxo-3-pyridazinyl)-;
(mit N,N-Dimethyl-bromacetamid):
2-Butyl-1-(2'-cyan-biphenylyl-4-methyl)-5-(1,4,5,6-tetrahydro-1-N,N-dimethylcarbamoylmethyl-4-methyl-6-oxo-3-pyridazinyl)-2-Butyl-1-(2'-cyan-biphenylyl-4-methyl)-6-(1,4,5,6-tetrahydro-1-N,N-dimethylcarbamoylmethyl-4-methyl-6-oxo-3-pyridazinyl)-.

## Beispiel 2

Zu einer Suspension von 2,84 g IIIa in 60 ml THF gibt man 2,8 g K-tert.-Butylat, rührt 30 Min. bei 20°, gibt 2,72 g IIb hinzu und rührt weitere 48 Std. bei 20°. Nach üblicher Aufarbeitung (gesättigte $NH_4Cl$-Lösung/Dichlormethan) erhält man 2-Butyl-5-[1-(2'-cyan-biphenylyl-4- methyl)-1,4,5,6-tetrahydro-4-methyl-6-oxo-3-pyridazinyl]-benzimidazol.

Analog erhält man mit IIa das 2-Butyl-5-[1,4,5,6-tetrahydro-1-(2'-methoxycarbonyl-biphenylyl-4-methyl)-4-methyl-6-oxo-3-pyridazinyl]-benzimidazol.

Analog erhält man aus 2-(4-Methoxyphenyl)-5-(1,4,5,6-tetrahydro-4-methyl-6-oxo-3-pyridazinyl)-benzimidazol die nachstehenden 2-(4-Methoxyphenyl)-benzimidazole:
mit IIb: 5-[1-(2'-Cyan-biphenylyl-4-methyl)-1,4,5,6-tetrahydro-4-methyl-6-oxo-3-pyridazinyl]-mit IIa: 5-[1-(2'-Methoxycarbonyl-biphenylyl-4-methyl)-1,4,5,6-tetrahydro-4-methyl-6-oxo-3-pyridazinyl]-.

## Beispiel 3

(a) Eine Lösung von 4,09 g 3-(3,4-Diaminophenyl)-1-(2'-cyan-biphenylyl-4-methyl)-1,4,5,6-tetrahydro-4-methylpyridazin-6-on ["D"; Öl; erhältlich aus 3-(3,4-Diaminophenyl)-1,4,5,6-tetrahydro-4-methyl-pyridazin-6-on und IIb in Gegenwart von K-tert.-Butylat in DMF bei 20°], 1,02 g Valeriansäure, 1,94 g N-(3-Dimethylaminopropyl)-N'-ethyl-carbodiimid, 1,40 g 1-Hydroxybenztriazol und 1,12 ml N-Methylmorpholin in 100 ml DMF wird 18 Std. bei 20° gerührt. Man gießt in gesättigte $Na_2CO_3$-Lösung, filtriert, löst den Rückstand in 135 ml Essigsäure und erhitzt 18 Std. auf 70°. Man dampft ein, chromatographiert den Rückstand an Kieselgel (Dichlormethan/Methanol 97:3) und erhält 2-Butyl-5- [1-(2'-cyan-biphenylyl-4-methyl)-1,4,5,6-tetrahydro-4-methyl-6-oxo-3-pyridazinyl]-benzimidazol, F. 70°.
Analog erhält man die folgenden 5-[1-(2'-Cyan-biphenylyl-4-methyl)-1,4,5,6-tetrahydro-4-methyl-6-oxo-3-pyridazinyl]-benzimidazole:
mit Essigsäure: 2-Methyl-
mit Propionsäure: 2-Ethyl-
mit Buttersäure: 2-Propyl-
mit Isovaleriansäure: 2-Isobutyl-
mit Hexansäure: 2-Pentyl-
mit Heptansäure: 2-Hexyl-
mit Benzoesäure: 2-Phenyl-
mit 4-Methoxybenzoesäure: 2-(4-Methoxyphenyl)-, F. 191°
mit Isonicotinsäure: 2-(4-Pyridyl)-mit 1-Methoxycarbonyl-indolin-5-carbonsäure:
2-(1-Methoxycarbonyl-5-indolinyl]-, FAB 595.
(b) Eine Suspension von 475 mg der nach (a) erhaltenen Verbindung und 620 mg Trimethylzinnazid in 50 ml Toluol wird 4 Tage gekocht und eingedampft. Der Rückstand wird an Kieselgel chromatographiert (Dichlormethan/Methanol 9:1). Man erhält 2-Butyl-5-[1-(2'-(1H-5-tetrazolyl)-biphenylyl-4-methyl)-1,4,5,6-tetrahydro-4-methyl-6-oxo-3-pyridazinyl]-benzimidazol, F. 137°.
Analog erhält man die folgenden 5-[1-(2'-(1H-5-Tetrazolyl)-biphenylyl-4-methyl)-1,4,5,6-tetrahydro-4-methyl-6-oxo-3-pyridazinyl]-benzimidazole:
2-Methyl-

2-Ethyl-
2-Propyl-
2-Isobutyl-
2-Pentyl-
2-Hexyl-
2-Phenyl-
2-(4-Methoxyphenyl)-, F. 150°
2-(4-Pyridyl)-
2-(1-Methoxycarbonyl-5-indolinyl)-, F. 209°.

**Beispiel 4**

Analog Beispiel 3 (a) erhält man aus 3-(3,4-Diaminophenyl)-1,4,5,6-tetrahydro-1-(4-methoxycarbonylbenzyl)-4-methyl-pyridazin-6-on [erhältlich aus 3-(3,4-Diaminophenyl)-1,4,5,6-tetrahydro-4-methyl-pyridazin-6-on und 4-Brommethyl-benzoesäuremethylester] und Valeriansäure das 2-Butyl-5-[1,4,5,6-tetrahydro-1-(4-methoxycarbonyl-benzyl)-4-methyl-6-oxo-3-pyridazinyl]-benzimidazol, FAB 433.

Analog erhält man aus 3-(3,4-Diaminophenyl)-1,4,5,6-tetrahydro-1-(2'-methoxycarbonyl-biphenylyl-4-methyl)-4-methyl-pyridazin-6-on [herstellbar aus 3-(3,4-Diaminophenyl)-1,4,5,6-tetrahydro-4-methyl-pyridazin-6-on und 4'-Brommethyl-biphenyl-2-carbonsäuremethylester] das 2-Butyl-5-[1,4,5,6-tetrahydro-1-(2'-methoxycarbonyl-biphenylyl-4-methyl)-4-methyl-6-oxo-3-pyridazinyl]-benzimidazol, FAB 509.

**Beispiel 5**

Eine Lösung von 0,30 g 1,3,5-Triazin und 4,09 g "D" (vgl. Bsp. 3 (a)) in 20 ml DMF wird 18 Std. auf 140° erhitzt. Man arbeitet wie üblich auf (Wasser/Ethylacetat; Chromatographie mit Ethylacetat/Methanol 95:5) und erhält 5-[1-(2'-Cyan-biphenylyl-4-methyl)-1,4,5,6-tetrahydro-4-methyl-6-oxo-3-pyridazinyl]-benzimidazol, FAB 420.

**Beispiel 6**

Ein Gemisch aus 3,89 g 1-p-Aminobenzyl-2-butyl-5-(1,4,5,6-tetrahydro-4-methyl-6-oxo-3-pyridazinyl)-benzimidazol [erhältlich durch Reaktion von 2-Butyl-5-(1,4,5,6-tetrahydro-4-methyl-6-oxo-3-pyridazinyl)-benzimidazol mit p-Nitrobenzylbromid zum 1-p-Nitrobenzylderivat und anschließende Hydrierung], 1,5 g Phthalsäureanhydrid und 40 ml $CHCl_3$ wird 16 Std. bei 20° gerührt. Nach üblicher Aufarbeitung erhält man 2-Butyl-1-[4-(2-carboxybenzamido)-benzyl]-5-(1,4,5,6-tetrahydro-4-methyl-6-oxo-3-pyridazinyl)-benzimidazol.

**Beispiel 7**

Ein Gemisch von 4,18 g 2-Butyl-1-(4-carboxybenzyl)-5-(1,4,5,6-tetrahydro-4-methyl-6-oxo-3-pyridazinyl)-benzimidazol [erhältlich durch Reaktion von IIIa mit 4-Brommethyl-benzoesäuremethylester zur 1-(4-Methoxycarbonylbenzyl)-Verbindung und nachfolgende Verseifung], 12 g $SOCl_2$ und 35 ml $CHCl_3$ wird 6 Std. gekocht und eingedampft. Das erhaltene rohe Säurechlorid wird durch mehrfaches Lösen in Toluol und Eindampfen von Resten $SOCl_2$ befreit und in 80 ml THF gelöst. Man tropft diese Lösung zu einer Lösung von 1,37 g Anthranilsäure und 0,8 g NaOH in 100 ml Wasser, rührt 24 Std. und säuert mit HCl bis pH 5 an. Nach üblicher Aufarbeitung erhält man 2-Butyl-1-[4-(2-carboxyanilino-carbonyl)-benzyl]-5-(1,4,5,6-tetrahydro-4-methyl-6-oxo-3-pyridazinyl)-benzimidazol.

**Beispiel 8**

Man löst 1 g 2-Butyl-5-[1,4,5,6-tetrahydro-4-methyl-6-oxo-1-(2'-(2-triphenylmethyl-2H-5-tetrazolyl)-biphenylyl-4-methyl)-3-pyridazinyl]-benzimidazol [erhältlich aus IIIa und 4-Brommethyl-2'-(2-triphenylmethyl-2H-5-tetrazolyl)-biphenyl] in 60 ml 4 N HCl in Dioxan und rührt 16 Std. bei 20°. Nach üblicher Aufarbeitung erhält man 2-Butyl-5-[1,4,5,6-tetrahydro-4-methyl-6-oxo-1-(2'-(1H-5-tetrazolyl)-biphenylyl-4-methyl)-3-pyridazinyl]-benzimidazol, F. 70°.

## Beispiel 9

Eine Lösung von 1 g 1-(2'-Benzyloxycarbonyl-biphenylyl-4-methyl)-5-(1-benzyloxycarbonyl-1,4,5,6-tetrahydro-4-methyl-6-oxo-3-pyridazinyl)-2-butyl-benzimidazol [erhältlich durch Reaktion von IIIa mit 4'-Brommethyl-biphenyl-2-carbonsäurebenzylester zu 1-(2'-Benzyloxycarbonyl-biphenylyl-4-methyl)-2-butyl-5-(1,4,5,6-tetrahydro-4-methyl-6-oxo-3-pyridazinyl)-benzimidazol und Umsetzung mit Bromessigsäurebenzylester] in 25 ml Isopropanol wird an 0,5 g 5%ig. Pd-Kohle bei 20° und 1 bar bis zur Aufnahme der berechneten $H_2$-Menge hydriert. Man filtriert ab, dampft ein und erhält 2-Butyl-1-(2'-carboxybiphenylyl-4-methyl)-5-(1-carboxymethyl-1,4,5,6-tetrahydro-4-methyl-6-oxo-3-pyridazinyl)-benzimidazol; F. 155°.

## Beispiel 10

Analog Beispiel 3 (b) erhält man aus den entsprechenden oben angegebenen (2'-Cyan-biphenylyl-4-methyl)-derivaten die nachstehenden 1-[2'-(1H-5-Tetrazolyl)-biphenylyl-4-methyl]-benzimidazole:

2-Butyl-5-(1,4,5,6-tetrahydro-1-methoxycarbonylmethyl-4-methyl-6-oxo-3-pyridazinyl)-
2-Butyl-6-(1,4,5,6-tetrahydro-1-methoxycarbonylmethyl-4-methyl-6-oxo-3-pyridazinyl)-
2-Butyl-5-(3,6-dihydro-2-oxo-2H-1,3,4-thiadiazin-5-yl)-
2-Butyl-6-(3,6-dihydro-2-oxo-2H-1,3,4-thiadiazin-5-yl)-
2-Butyl-5-(3,6-dihydro-1-methoxycarbonylmethyl-2-oxo-2H-1,3,4-thiadiazin-5-yl)-
2-Butyl-6-(3,6-dihydro-1-methoxycarbonylmethyl-2-oxo-2H-1,3,4-thiadiazin-5-yl)-
2-Butyl-5-(3,6-dihydro-6-methyl-2-oxo-2H-1,3,4-thiadiazin-5-yl)-
2-Butyl-6-(3,6-dihydro-6-methyl-2-oxo-2H-1,3,4-thiadiazin-5-yl)-
2-Butyl-5-(1,4,5,6-tetrahydro-1-o-methoxycarbonylbenzyl-4-methyl-6-oxo-3-pyridazinyl)-
2-Butyl-6-(1,4,5,6-tetrahydro-1-o-methoxycarbonylbenzyl-4-methyl-6-oxo-3-pyridazinyl)-
2-Butyl-5-(1,4,5,6-tetrahydro-1-N,N-dimethylcarbamoylmethyl-4-methyl-6-oxo-3-pyridazinyl)-
2-Butyl-6-(1,4,5,6-tetrahydro-1-N,N-dimethylcarbamoylmethyl-4-methyl-6-oxo-3-pyridazinyl)-, FAB 604,

sowie die nachfolgenden [1,4,5,6-Tetrahydro-4-methyl-6-oxo-1-(2'-(1H-5-tetrazolyl)-biphenylyl-4-methyl)-3-pyridazinyl]-benzimidazole:

5-, F. 175°
2-Butyl-1-methoxycarbonylmethyl-5-
2-Butyl-1-methoxycarbonylmethyl-6-.

## Beispiel 11

Analog Beispiel 1 (c) erhält man durch Verseifung der entsprechenden oben angegebenen Methylester die nachstehenden Benzimidazole:

2-Butyl-5-[1-(4-carboxybenzyl)-1,4,5,6-tetrahydro-4-methyl-6-oxo-3-pyridazinyl]-, F. 134°; FAB 418
2-Butyl-5-[1-(2'-carboxy-biphenylyl-4-methyl)-1,4,5,6-tetrahydro-4-methyl-6-oxo-3-pyridazinyl]-, FAB 495; Tetrahydrat, F. 145°
2-Butyl-5-(1-carboxymethyl-1,4,5,6-tetrahydro-4-methyl-6-oxo-3-pyridazinyl)-1-[2'-(1H-5-tetrazolyl)-biphenylyl-4-methyl]-, F. > 300°, FAB 577
2-Butyl-6-(1-carboxymethyl-1,4,5,6-tetrahydro-4-methyl-6-oxo-3-pyridazinyl)-1-[2'-(1H-5-tetrazolyl)-biphenylyl-4-methyl]- , FAB 577
2-Butyl-5-(1-carboxymethyl-3,6-dihydro-2-oxo-2H-1,3,4-thiadiazin-5-yl)-1-[2'-(1H-5-tetrazolyl)-biphenylyl-4-methyl]-
2-Butyl-6-(1-carboxymethyl-3,6-dihydro-2-oxo-2H-1,3,4-thiadiazin-5-yl)-1-[2'-(1H-5-tetrazolyl)-biphenylyl-4-methyl]-
2-Butyl-1-carboxymethyl-5-[1,4,5,6-tetrahydro-4-methyl-6-oxo-1-(2'-(1H-5-tetrazolyl)-biphenylyl-4-methyl)]-3-pyridazinyl-
2-Butyl-1-carboxymethyl-6-[1,4,5,6-tetrahydro-4-methyl-6-oxo-1-(2'-(1H-5-tetrazolyl)-biphenylyl-4-methyl)]-3-pyridazinyl-
2-Butyl-5-(1-o-carboxybenzyl-1,4,5,6-tetrahydro-4-methyl-6-oxo-3-pyridazinyl)-1-[2'-(1H-5-tetrazolyl)-biphenylyl-4-methyl]-
2-Butyl-6-(1-o-carboxybenzyl-1,4,5,6-tetrahydro-4-methyl-6-oxo-3-pyridazinyl)-1-[2'-(1H-5-tetrazolyl)-biphenylyl-4-methyl]-.

**Beispiel 12**

Eine Lösung von 552 mg 2-Butyl-1-(2'-carboxy-biphenylyl-4-methyl)-5-(1-carboxymethyl-1,4,5,6-tetrahydro-4-methyl-6-oxo-3-pyridazinyl)-benzimidazol und 240 mg Na-3-nitrobenzolsulfonat in 5,5 ml Wasser und 4,5 ml 1 N NaOH wird 1 Std. gekocht und eingedampft. Chromatographie des Rückstands an Kieselgel (Ethylacetat/Methanol 1:1) gibt 2-Butyl-1-(2'-carboxy-biphenylyl-4-methyl)-5-(1-carboxymethyl-1,6-dihydro-4-methyl-6-oxo-3-pyridazinyl)-benzimidazol; FAB 551; F. > 300°,
Analog erhält man durch Dehydrierung der entsprechenden 1,4,5,6-Tetrahydro-6-oxo-3-pyridazinyl-derivate die nachstehenden Benzimidazole:
2-Butyl-1-(2'-carboxy-biphenylyl-4-methyl)-6-(1-carboxymethyl-1,6-dihydro-4-methyl-6-oxo-3-pyridazinyl)-, FAB 551; F. > 300°;
2-Butyl-5-[1-(2'-cyan-biphenylyl-4-methyl)-1,6-dihydro-4-methyl-6-oxo-3-pyridazinyl]-, FAB 474; Trihydrat, F. 238°;
2-Butyl-5-[1,6-dihydro-4-methyl-6-oxo-1-(2'-(1H-5-tetrazolyl)-biphenylyl-4-methyl)-3-pyridazinyl]-
5-[1,6-Dihydro-4-methyl-6-oxo-1-(2'-(1H-5-tetrazolyl)-biphenylyl-4-methyl)-3-pyridazinyl]-2-p-methoxyphenyl-
5-[1,6-Dihydro-4-methyl-6-oxo-1-(2'-(1H-5-tetrazolyl)-biphenylyl-4-methyl)-3-pyridazinyl]-
2-(2,3-Dihydro-1-methoxycarbonyl-5-indolyl)-5-[1,6-dihydro-4-methyl-6-oxo-1-(2'-(1H-5-tetrazolyl)-biphenylyl-4-methyl)-3-pyridazinyl]-
2-Butyl-5-[1-(2'-carboxy-biphenylyl-4-methyl)-1,6-dihydro-4-methyl-6-oxo-3-pyridazinyl]-, F. 168°
2-Butyl-5-[1-(4-carboxybenzyl)-1,6-dihydro-4-methyl-6-oxo-3-pyridazinyl]-, FAB 416; Pentahydrat, F. 158°
2-Butyl-5-(1-carboxymethyl-1,6-dihydro-4-methyl-6-oxo-3-pyridazinyl)-1-[2'-(1H-5-tetrazolyl)-biphenylyl-4-methyl]-
2-Butyl-6-(1-carboxymethyl-1,6-dihydro-4-methyl-6-oxo-3-pyridazinyl)-1-[2'-(1H-5-tetrazolyl)-biphenylyl-4-methyl]-, F. > 300°; FAB 575
2-Butyl-1-carboxymethyl-5-[1,6-dihydro-4-methyl-6-oxo-1-[2'-(1H-5-tetrazolyl)-biphenylyl-4-methyl)]-
2-Butyl-1-carboxymethyl-6-[1,6-dihydro-4-methyl-6-oxo-1-[2'-(1H-5-tetrazolyl)-biphenylyl-4-methyl)]-
2-Butyl-5-(1-o-carboxybenzyl-1,6-dihydro-4-methyl-6-oxo-3-pyridazinyl)-1-[2'-(1H-5-tetrazolyl)-biphenylyl-4-methyl]-
2-Butyl-6-(1-o-carboxybenzyl-1,6-dihydro-4-methyl-6-oxo-3-pyridazinyl)-1-[2'-(1H-5-tetrazolyl)-biphenylyl-4-methyl]-
2-Butyl-5-(1-N,N-dimethylcarbamoylmethyl-1,6-dihydro-4-methyl-6-oxo-3-pyridazinyl)-1-[2'-(1H-5-tetrazolyl)-biphenylyl-4-methyl]-
2-Butyl-6-(1-N,N-dimethylcarbamoylmethyl-1,6-dihydro-4-methyl-6-oxo-3-pyridazinyl)-1-[2'-(1H-5-tetrazolyl)-biphenylyl-4-methyl]-.

**Beispiel 13**

Eine Lösung von 4,09 g "D" (vgl. Beispiel 3 (a)), 1,7 g Anisaldehyd und 2,5 g $Na_2S_2O_5$ in 60 ml DMF wird 3 Std. gekocht. Man versetzt mit Wasser. Der Niederschlag wird abfiltriert und getrocknet. Man erhält 5-[1-(2'-Cyan-biphenylyl-4-methyl)-1,4,5,6-tetrahydro-4-methyl-6-oxo-3-pyridazinyl]-2-(4-methoxyphenyl)-benzimidazol, F. 191°; FAB 526.

Analog erhält man aus "D" und 1-Methoxycarbonyl-5-formylindolin (F. 103°; herstellbar durch Reaktion von Indolin mit Chlorameisensäuremethylester in Dichlormethan in Gegenwart von Pyridin zu 1-Methoxycarbonylindolin und nachfolgende Umsetzung mit 1,1-Dichlormethyl-methylether/$TiCl_4$ in Dichlormethan) das 5-[1-(2'-Cyan-biphenylyl-4-methyl)-1,4,5,6-tetrahydro-4-methyl-6-oxo-3-pyridazinyl]-2-(1-methoxycarbonyl-5-indolinyl)-benzimidazol, FAB 595.

**Beispiel 14**

a) Analog Beispiel 1 a), jedoch in DMF (statt THF), erhält man aus 2-Cyclopropyl-5-(1,4,5,6-tetrahydro-4-methyl-6-oxo-3-pyridazinyl)-benzimidazol [F. 287°; erhältlich durch Reaktion von 3-(3,4-Diaminophenyl)-1,4,5,6-tetrahydro-4-methyl-6-pyridazinon mit Cyclopropancarbonsäure und nachfolgende Cyclisierung mit Essigsäure analog Beispiel 3] und II b nach üblicher Aufarbeitung (Ethylacetat; Chromatographie an Kieselgel mit Ethylacetat/Methanol 96:4) zunächst das 1-(2'-Cyan-biphenylyl-4-methyl)-2-cyclopropyl-6-(1,4,5,6-tetrahydro-4-methyl-6-oxo-3-pyridazinyl)-benzimidazol (Rf 0,50), dann das 1-(2'-Cyan-biphenylyl-4-methyl)-2-cyclopropyl-5-(1,4,5,6-tetrahydro-4-methyl-6-oxo-3-pyridazinyl)-benzimidazol (Rf 0,65; beides gelbliche Öle).

b) Analog Beispiel 3 b) erhält man aus den beiden vorstehend genannten Verbindungen mit Trimethylzinnazid das 2-Cyclopropyl-6-(1,4,5,6-tetrahydro-4-methyl-6-oxo-3-pyridazinyl)-1-[2'-(1H-5-tetrazolyl)-biphenylyl-4-methyl]-benzimidazol(Dihydrat, F. 216°) sowie das 2-Cyclopropyl-5-(1,4,5,6-tetrahydro-4-methyl-6-oxo-3-pyridazinyl)-1-[2'-(1H-5-tetrazolyl)-biphenylyl-4-methyl]-benzimidazol (Monohydrat, F. 265°).

c) Analog Beispiel 12 erhält man aus den beiden vorstehend genannten Verbindungen mit Na-3-nitrobenzolsulfonat (4 Std. Kochen in 0,5 N wäßriger NaOH) das 2-Cyclopropyl-6-(1,6-dihydro-4-methyl-6-oxo-3-pyridazinyl-1-[2'-(1H-5-tetrazolyl)-biphenylyl-4-methyl]-benzimidazol (Monohydrat, F. 225°) sowie das 2-Cyclopropyl-5-(1,6-dihydro-4-methyl-6-oxo-3-pyridazinyl)-1-[2'-(1H-5-tetrazolyl)-biphenylyl-4-methyl]-benzimidazol (Tetrahydrat, F. 260°).

## Beispiel 15

a) Analog Beispiel 1 a), jedoch in DMF (statt THF), erhält man aus 2-Cyclopropylmethyl-5-(1,4,5,6-tetrahydro-4-methyl-6-oxo-3-pyridazinyl)-benzimidazol [erhältlich durch Reaktion von 3-(3,4-Diaminophenyl)-1,4,5,6-tetrahydro-4-methyl-6-pyridazinon mit Cyclopropylessigsäure und nachfolgende Cyclisierung mit Essigsäure analog Beispiel 3] und II b nach üblicher Aufarbeitung (Ethylacetat; Chromatographie an Kieselgel mit Ethylacetat/Methanol 96:4) zunächst das 1-(2'-Cyan-biphenylyl-4-methyl)-2-cyclopropylmethyl-6-(1,4,5,6-tetrahydro-4-methyl-6-oxo-3-pyridazinyl)-benzimidazol, dann das 1-(2'-Cyan-biphenylyl-4-methyl)-2-cyclopropylmethyl-5-(1,4,5,6-tetrahydro-4-methyl-6-oxo-3-pyridazinyl)-benzimidazol.

b) Analog Beispiel 3 b) erhält man aus den beiden vorstehend genannten Verbindungen mit Trimethylzinnazid das 2-Cyclopropylmethyl-6-(1,4,5,6-tetrahydro-4-methyl-6-oxo-3-pyridazinyl)-1-[2'-(1H-5-tetrazolyl)-biphenylyl-4-methyl]-benzimidazol sowie das 2-Cyclopropylmethyl-5-(1,4,5,6-tetrahydro-4-methyl-6-oxo-3-pyridazinyl)-1-[2'-(1H-5-tetrazolyl)-biphenylyl-4-methyl]-benzimidazol.

c) Analog Beispiel 12 erhält man aus den beiden vorstehend genannten Verbindungen mit Na-3-nitrobenzolsulfonat (4 Std. Kochen in 0,5 N wäßriger NaOH) das 2-Cyclopropylmethyl-6-(1,6-dihydro-4-methyl-6-oxo-3-pyridazinyl)-1-[2'-(1H-5-tetrazolyl)-biphenylyl-4-methyl]-benzimidazol sowie das 2-Cyclopropylmethyl-5-(1,6-dihydro-4-methyl-6-oxo-3-pyridazinyl)-1-[2'-(1H-5-tetrazolyl)-biphenylyl-4-methyl]-benzimidazol.

## Beispiel 16

a) Analog Beispiel 1 a) erhält man aus den beiden in Beispiel 14 b) genannten Verbindungen durch Reaktion mit den nachstehenden Verbindungen der Formel $R^3$-X die nachstehenden 2-Cyclopropyl-1-[2'-(1H-5-tetrazolyl)-biphenylyl-4-methyl]-benzimidazole:
mit Bromessigsäuremethylester:
-5-(1,4,5,6-tetrahydro-1-methoxycarbonylmethyl-4-methyl-6-oxo-3-pyridazinyl)--6-(1,4,5,6-tetrahydro-1-methoxycarbonylmethyl-4-methyl-6-oxo-3-pyridazinyl)-, Dihydrat, F. 125°;
mit N,N-Dimethyl-chloracetamid:
-5-(1,4,5,6-tetrahydro-1-N,N-dimethyl-carbamoylmethyl-4-methyl-6-oxo-3-pyridazinyl)-
-6-(1,4,5,6-tetrahydro-1-N,N-dimethyl-carbamoylmethyl-4-methyl-6-oxo-3-pyridazinyl)-, FAB 588;
mit Benzylbromid:
-5-(1-benzyl-1,4,5,6-tetrahydro-4-methyl-6-oxo-3-pyridazinyl)-
-6-(1-benzyl-1,4,5,6-tetrahydro-4-methyl-6-oxo-3-pyridazinyl)-
mit 2-Brommethyl-benzoesäuremethylester:
-5-[1,4,5,6-tetrahydro-1-(2-methoxycarbonyl-benzyl)-4-methyl-6-oxo-3-pyridazinyl]-
-6-[1,4,5,6-tetrahydro-1-(2-methoxycarbonyl-benzyl)-4-methyl-6-oxo-3-pyridazinyl]-, FAB 651

b) Analog Beispiel 12 erhält man durch Dehydrierung der vorstehend genannten Vebindungen mit Na-3-nitrobenzolsulfonat in wäßriger NaOH-Lösung die nachstehenden 2-Cyclopropyl-1-[2'-(1H-5-tetrazolyl)-biphenylyl-4-methyl]-benzimidazole (wobei Estergruppen verseift werden!):
-5-(1-carboxymethyl-1,6-dihydro-4-methyl-6-oxo-3-pyridazinyl)-,
-6-(1-carboxymethyl-1,6-dihydro-4-methyl-6-oxo-3-pyridazinyl)-,FAB 559; Trihydrat, F. > 300°
-5-(1,6-dihydro-1-N,N-dimethyl-carbamoylmethyl-4-methyl-6-oxo-3-pyridazinyl)-
-6-(1,6-dihydro-1-N,N-dimethyl-carbamoylmethyl-4-methyl-6-oxo-3-pyridazinyl)-, FAB 586
-5-(1-benzyl-1,6-dihydro-4-methyl-6-oxo-3-pyridazinyl)-
-6-(1-benzyl-1,6-dihydro-4-methyl-6-oxo-3-pyridazinyl)-
-5-[1-(2-carboxybenzyl)-1,6-dihydro-4-methyl-6-oxo-3-pyridazinyl]-

-6-[1-(2-carboxybenzyl)-1,6-dihydro-4-methyl-6-oxo-3-pyridazinyl]-, F. > 300°, FAB 636.

Beispiel 17

(a) Analog Beispiel 1 (a), jedoch in DMF, erhält man aus 2-Cyclopropyl-5-(1,4,5,6-tetrahydro-6-oxo-3-pyridazinyl)-benzimidazol [erhältlich durch Reaktion von 3-(3,4-Diaminophenyl)-1,4,5,6-tetrahydro-6-pyridazinon mit Cyclopropancarbonsäure und nachfolgende Cyclisierung mit Essigsäure analog Beispiel 3] und IIb nach üblicher Aufarbeitung (Chromatographie an Kieselgel mit Ethylacetat/Methanol 96:4) zunächst das 1-(2'-Cyan-biphenylyl-4-methyl)-2-cyclopropyl-6-(1,4,5,6-tetrahydro-6-oxo-3-pyridazinyl)-benzimidazol (FAB 446), dann das 1-(2'-Cyan-biphenylyl-4-methyl)-2-cyclopropyl-5-(1,4,5,6-tetrahydro-6-oxo-3-pyridazinyl)-benzimidazol (FAB 446).
(b) Analog Beispiel 3 (b) erhält man aus den beiden vorstehend genannten Verbindungen mit Trimethylzinnazid das 2-Cyclopropyl-6-(1,4,5,6-tetrahydro-6-oxo-3-pyridazinyl)-1-[2'-(1H-5-tetrazolyl)-biphenylyl-4-methyl)-benzimidazol sowie das 2-Cyclopropyl-5-(1,4,5,6-tetrahydro-6-oxo-3-pyridazinyl-1-(2'-(1H-5-tetrazolyl)-biphenylyl-4-methyl]-benzimidazol.

Beispiel 18

(a) Analog Beispiel 1 (a), jedoch in DMF, erhält man aus 2-Cyclopropyl-5-(1,4,5,6-tetrahydro-4, 4-dimethyl-6-oxo-3-pyridazinyl)-benzimidazol [erhältlich durch Reaktion von 3-(3,4-Diaminophenyl)-1,4,5,6-tetrahydro-4,4-dimethyl-6-pyridazinon mit Cyclopropancarbonsäure und nachfolgende Cyclisierung mit Essigsäure analog Beispiel 3] und IIb nach üblicher Aufarbeitung (Chromatographie an Kieselgel mit Ethylacetat/Methanol 96:4) zunächst das 1-(2'-Cyan-biphenylyl-4-methyl)-2-cyclopropyl-6-(1,4,5,6-tetrahydro-4,4-dimethyl-6-oxo-3-pyridazinyl)-benzimidazol (FAB 474), dann das 1-(2'-Cyan-biphenylyl-4-methyl)-2-cyclopropyl-5-(1,4,5,6-tetrahydro-4,4-dimethyl-6-oxo-3-pyridazinyl)-benzimidazol (FAB 474).
(b) Analog Beispiel 3 (b) erhält man aus den beiden vorstehend genannten Verbindungen mit Trimethylzinnazid das 2-Cyclopropyl-6-(1,4,5,6-tetrahydro-4,4-dimethyl-6-oxo-3-pyridazinyl)-1-[2'-(1H-5-tetrazolyl)-biphenylyl4-methyl]-benzimidazol (F. 237°) sowie das 2-Cyclopropyl-5-(1,4,5,6-tetrahydro-4,4-dimethyl-6-oxo-3-pyridazinyl)-1-[2'-(1H-5-tetrazolyl)-biphenylyl-4-methyl]-benzimidazol (F. 248°).

Die nachfolgenden Beispiele betreffen pharmazeutische Zubereitungen, die Wirkstoffe der Formel I oder ihre Salze enthalten.

**Beispiel A: Tabletten und Dragees**

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

**Beispiel B: Hartgelatine-Kapseln**

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

**Beispiel C: Weichgelatine-Kapseln**

übliche Weichgelatine-Kapseln werden mit einem Gemisch aus jeweils 50 mg Wirkstoff und 250 mg Olivenöl gefüllt.

**Beispiel D: Ampullen**

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

**Beispiel E: Wässerige Suspension für orale Applikation**

Eine wässerige Suspension wird in üblicher Weise hergestellt. Die Einheitsdosis (5 ml) enthält 100 mg Wirkstoff, 100 mg Na-Carboxymethylcellulose, 5 mg Na-Benzoat und 100 mg Sorbit.

**Patentansprüche**

**1.** Benzimidazolderivate der Formel I

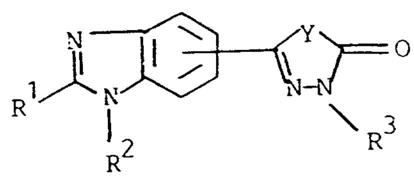

I

worin

$R^1$ H, A, $C_pH_{2p}$-($C_{3-7}$-Cycloalkyl), OA, SA, Ar oder $Het^1$,

$R^2$ und $R^3$ jeweils

(1)

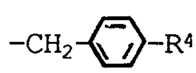

(2)

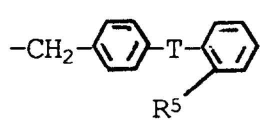

(3)

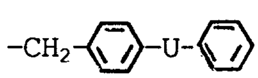

(4) -$C_{1-10}$-Alkyl, -$C_nH_{2n}$-COOR, -$C_nH_{2n}$-CN, -$C_mH_{2m}$-Ar, -$C_mH_{2m}$-$Het^2$, -$C_nH_{2n}$-1H-5-Tetrazolyl, $C_nH_{2n}$-CH=CH-Ar, -$C_nH_{2n}$-CO-N$(R)_2$, -$C_mH_{2m}$-CO-R, -$C_mH_{2m}$-CO-Ar, $C_nH_{2n}$-O-CON$(R)_2$ oder -$C_nH_{2n}$-NR-CO-N$(R)_2$, oder (5) H,

Y -C$(R)_2$-C$(R)_2$-, -CR=CR- oder -C$(R)_2$-S-,

die Reste R unabhängig voneinander H oder A,

$R^4$ COOR, CN oder 1H-5-Tetrazolyl,

$R^5$ COOR, CN, $NO_2$, $NH_2$, $NHCOCF_3$, $NHSO_2CF_3$ oder 1H-5-Tetrazolyl,

T fehlt, -NR-CO-, -CO-NR- oder -CH=CH-,

U -CH=C(COOR)-, -CH=C(CN)-, -CH=C(1H-5-Tetrazolyl)-, -O-CH(COOR)- oder -NR-CH-(COOR)-,

m und p jeweils 0, 1, 2, 3, 4, 5, 6, 7, 8, 9 oder 10,

n 1, 2, 3, 4, 5, 6, 7, 8, 9 oder 10,

A Alkyl mit 1-6 C-Atomen,

Ar eine unsubstituierte oder eine ein- oder zweifach durch A, Hal, $CF_3$, OH, OA, COOH, COOA, CN, $NO_2$, $NH_2$, NHA und/oder $N(A)_2$ substituierte Phenyl- oder Naphthylgruppe,

$Het^1$ eine unsubstituierte oder eine durch A, $C_{1-7}$-Alkanoyl oder COOA monosubstituierte Indolyl-, 2,3-Dihydroindolyl-, Benzofuryl-, 2,3-Dihydrobenzofuryl-, Benzothienyl- oder 2,3-Dihydrobenzothienyl-gruppe,

$Het^2$ einen unsubstituierten oder durch A substituierten fünf- oder sechsgliedrigen heteroaromatischen Rest mit 1 bis 3 N-, O- und/oder S-Atomen, der auch mit einem Benzol- oder Pyridinring kondensiert sein kann und

Hal F, Cl, Br oder I bedeuten,

mit der Maßgabe, daß

1. Y nur dann $-C(R)_2-C(R)_2-$ oder -CR=CR- bedeutet, wenn $R^1$ von $-C_pH_{2p}-(C_{3-7}\text{-Cycloalkyl})$ und/oder wenn $R^3$ von H verschieden ist,

2. mindestens einer der Reste $R^2$ und $R^3$ eine der Bedeutungen (1), (2) oder (3) hat,

sowie ihre Salze.

**2.**

a) 2-Butyl-5-[1-(2'-carboxy-biphenylyl-4-methyl)-1,6-dihydro-4-methyl-6-oxo-3-pyridazinyl]-benzimidazol;

b) 2-Butyl-1-(2'-carboxy-biphenylyl-4-methyl)-6-(1-carboxymethyl-1,4,5,6-tetrahydro-4-methyl-6-oxo-3-pyridazinyl)-benzimidazol

c) 2-Butyl-1-(2'-carboxy-biphenylyl-4-methyl)-5-(1-carboxymethyl-1,4,5,6-tetrahydro-4-methyl-6-oxo-3-pyridazinyl)-benzimidazol.

**3.** Verfahren zur Herstellung von Benzimidazolderivaten der Formel I nach Anspruch 1 sowie ihrer Salze, dadurch gekennzeichnet, daß man

(a) eine Verbindung der Formel II

E-Z II

worin

E Cl, Br, I oder eine freie oder reaktionsfähig funktionell abgewandelte OH-Gruppe und

Z $R^2$ oder $R^3$, jedoch nicht H, bedeuten,

mit einer Verbindung der Formel III

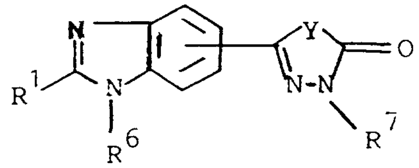

III

worin

$R^6$ $R^2$ (falls Z $R^3$ ist) und

$R^7$ $R^3$ (falls Z $R^2$ ist) bedeuten,

mindestens einer der Reste $R^6$ und $R^7$ jedoch H bedeutet und

$R^1$ und Y die in Anspruch 1 angegebene Bedeutung haben,

umsetzt,

oder

(b) eine Verbindung der Formel IV

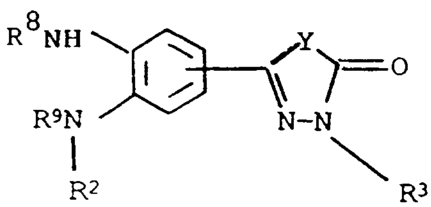

IV

worin

$R^8$ $R_1$-CO oder H, und

$R^9$ H (falls $R^8$ $R^1$-CO ist) oder $R^1$-CO (falls $R^8$ H ist)

bedeuten,

und $R^1$, $R^2$, $R^3$ und Y die in Anspruch 1 angegebenen Bedeutungen haben,
mit einem cyclisierenden Mittel behandelt,
oder

(c) zur Herstellung einer Verbindung der Formel I, worin T -NR-CO- oder -CO-NR- bedeutet, eine Verbindung der Formel V

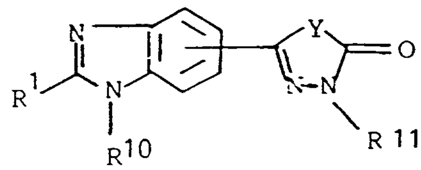

V

worin

$R^{10}$ $R^2$ oder

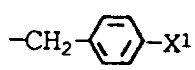

$R^{11}$ $R^3$ (falls

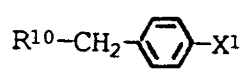

bedeutet) oder

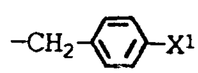

(falls $R^{10}$ $R^2$ bedeutet), und

$X^1$ $NH_2$ oder COOH bedeuten, und

$R^1$ und Y die in Anspruch 1 angegebenen Bedeutungen haben,

oder ein reaktionsfähiges Derivat dieser Verbindung mit einer Verbindung der Formel VI

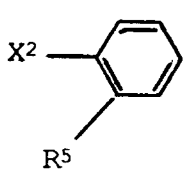

VI

worin

$X^2$ COOH (falls $X^1$ $NH_2$ ist) oder $NH_2$ (falls $X^1$ COOH ist) bedeutet und

$R^5$ die in Anspruch 1 angegebene Bedeutung hat,

oder mit einem reaktionsfähigen Derivat dieser Verbindung umsetzt,

oder daß man eine Verbindung der Formel I aus einem ihrer funktionellen Derivate durch Behandeln mit einem solvolysierenden oder hydrogenolysierenden Mittel in Freiheit setzt,

und/oder daß man in einer Verbindung der Formel I einen oder mehrere Rest(e) $R^2$ und/oder $R^3$ in einen oder mehrere andere Reste $R^2$ und/oder $R^3$ umwandelt und/oder eine Verbindung der Formel I, worin Y -CHR-CHR- bedeutet, durch Behandeln mit einem dehydrierenden Mittel in eine Verbindung der Formel I, worin Y -CR=CR- bedeutet, überführt und/oder eine Base oder Säure der Formel I in eines ihrer Salze umwandelt.

**4.** Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

**5.** Pharmazeutische Zubereitung, gekennzeichnet durch einen Gehalt an mindestens einer Verbindung der Formel I nach Anspruch 1 und/oder einem ihrer physiologisch unbedenklichen Salze.

**6.** Verbindung der Formel I nach Anspruch 1 und ihre physiologisch unbedenklichen Salze zur Bekämpfung von Krankheiten.

**7.** Verwendung von Verbindungen der Formel I nach Anspruch 1 und/oder ihrer physiologisch unbedenklichen Salze zur Herstellung eines Arzneimittels.

**8.** Verwendung von Verbindungen der Formel I nach Anspruch 1 und/oder ihrer physiologisch unbedenklichen Salze bei der Bekämpfung von Krankheiten.